# EUROPEAN PATENT APPLICATION

(11) **EP 2 471 388 A1**
(43) Date of publication of application: **04.07.2012**
(21) Application number: 12161528.0
(22) Date of filing: 10.12.2008
(51) Int. Cl.: A23L 1/308, A23L 1/29, A23L 2/52, A23C 9/13, A61K 31/702, A61K 31/721, A61K 31/733, A61P 1/12, A61P 1/10, A61P 39/00

(54) **Paediatric fibre mixture**

(30) Priority: 10.12.2007 WO PCT/NL2007/050639
(62) Divisional of application: 08858418.0
(71) Applicant: N.V. Nutricia, 2712 HM Zoetermeer (NL)
(72) Inventor: Goedhart, Anna Christina, 3043 PR Rotterdam (NL); Alles, Martine Sandra, 7328 NH Apeldoorn (NL); van Laere, Katrien Maria Jozefa, 6666 WS Heteren (NL)
(74) Representative: Swinkels, Bart Willem

(57) **Abstract**

The present invention relates to a liquid nutritional composition for paediatric patients comprising beta-galacto-oligosaccharides, fructan, non-digestible alpha-glucan and hemicellulose.

## Description

### FIELD OF THE INVENTION

The present invention is in the field of liquid enteral nutrition.

### BACKGROUND OF THE INVENTION

Many patients, including paediatric patients, rely for their food intake partly or totally on clinical nutrition. Typical clinical enteral nutritional products are oral nutrition supplements and enteral tube feedings. Suitable oral supplements include ready-to-drink milk or yoghurt based sip feeds and high energy sip feeds. These liquid enteral clinical nutritional products are usually designed to be nutritionally complete, i.e. provide on a daily basis all the macro- and micronutrients needed for optimal growth and development and preservation of lean tissue mass and body composition. Clinical nutrition for the paediatric population is especially designed for this age group and it differs in its composition from clinical nutrition designed for adult patients, because of the different needs of paediatric patients compared with adult patients. For example, adult feeds contain too much protein, sodium, potassium, chloride and magnesium, and inappropriate vitamin and trace element prophiles to meet the needs of paediatric patients.

The fibre mixtures used in enteral nutrition for paediatric patients used so far are mostly based on fibre mixtures suitable for adults.

Paediatric patients receiving clinical enteral nutrition often suffer from constipation. Constipation is in paediatric subjects often a problem due to their lesser physical activity. Lactulose or poly-ethylene glycol are commonly prescribed laxatives in case of chronic childhood constipation. However, these are fibres not naturally occurring in food, having their effect mainly in the proximal colon and having no additional benefits.

EP 0756828 describes a fiber mix with a composition representing the dietary fiber in a typical adult Western diet. WO 2005/039319 discloses a preparation comprising *Bifidobacterium breve* and a mixture of non-digestible carbohydrates for infants. EP1597979 discloses a synergistic effect between polyfructose and galactooligosaccharides. WO2006/046871 discloses the use of *L. rhamnosus* for the preparation of nutritional compositions. WO 02/051264 discloses the use of non-digestible oligosaccharides with terminal arabinose unit for an anti-adhesion effect and a bifidogenic effect.

### SUMMARY OF THE INVENTION

The present inventors found that paediatric tube and sip feeds often do not contain qualitatively and quantitatively sufficient fibre. It thus was an object to provide an optimal fibre mixture to be administered in a liquid enteral nutritional composition to paediatric patients and/or constipated children.

The present invention thus relates to a fibre mixture designed especially for clinical enteral nutrition (oral nutrition supplements and enteral tube feeding) for children of 1 - 14 years, particularly paediatric patients. The mixture comprises beta-galacto-oligosaccharides, fructan, non-digestible alpha-gluco-polysaccharides, and hemicellulose.

It was found in a clinical study with children suffering from chronic constipation that this specific combination of different fibres effectively reduced constipation. This is especially advantageous for paediatric patients that suffer more from constipation than healthy children due to their lesser physical activity or due to their disease. Compared with lactulose, a prolonged improved softening of stool, occurring slower and more steady, was observed with this fibre mixture. The especially designed fibre mixture resulted in a prolonged fermentation throughout the colon, including the distal column, without a reduction in water content. This is especially advantageous for paediatric patients who rely for both water and nutritional intake solely on tube and/or sip feeds. Also the taste of the fibre drink was improved compared to a lactulose comprising drink.

Compared with fibre mixtures known in the art for infant formulae, which results in the formation of high amounts of lactate and acetate and low amounts of propionate and butyrate by colonic fermentation, and with fibre mixtures known in the art for adult nutrition, which result in the formation of low amounts of lactate and higher amounts of propionate by colonic fermentation, the fibre mixtures of the present invention showed an intermediate effect on lactate, acetate, propionate and butyrate formation. The fibre mixtures of the present invention are therefore more suitable for paediatric patients, having a colonic fermentation pattern in between those of infants and adults.

Despite the presence of insoluble fibres (the hemicellulose and the non-digestible alpha-glucan) the liquid clinical enteral products of the present invention are stable and are suitable to be administered via a straw or a tube.

### DETAILED DESCRIPTION OF THE INVENTION

The present invention relates to a method for providing nutritional support to a subject in need thereof and/or for providing nutrition to a subject and/or for treatment and/or prevention in a subject of one or more selected form the group consisting of constipation, disease-related malnutrition, inflammation, diarrhoea and infections, said method comprising administering a composition comprising a mixture of non-digestible carbohydrates, wherein the mixture of non-digestible carbohydrates comprises a) beta-galacto-oligosaccharide; b) fructan; c) non-digestible alpha-glucan; and d) hemicellulose to said subject.

In other words the present invention relates to the use of a mixture of non-digestible carbohydrates, wherein the mixture of non-digestible carbohydrates comprises a) beta-galacto-oligosaccharide; b) fructan; c) non-digestible alpha-glucan; and d) hemicellulose, for the manufacture of a liquid nutritional composition for administration to children of 1 to 14 years old. In one embodiment said liquid nutritional composition is for one selected from the group consisting of administration to children in need of nutrition, administration to children needing nutritional support, prevention and/or treatment of disease-related malnutrition, prevention and/or treatment of inflammation, diarrhoea and/or infections.

The present invention can also be worded as a liquid nutritional composition comprising a mixture of non-digestible carbohydrates, wherein the mixture of non-digestible carbohydrates comprises a) beta-galacto-oligosaccharide; b) fructan; c) non-digestible alpha-glucan; and d) hemicellulose for use in, in particular administration to, children of 1 to 14 years old. In one embodiment said liquid nutritional composition comprising said mixture of non-digestible carbohydrates is for one selected form administration to children in need of nutrition, administration to children needing nutritional support, prevention and/or treatment of disease-related malnutrition, prevention and/or treatment of inflammation, diarrhoea and/or infections.

In one embodiment said liquid nutritional composition comprising said mixture of non-digestible carbohydrates is for prevention and/or treatment of constipation. In one embodiment said liquid nutritional composition comprising said mixture of non-digestible carbohydrates is for paediatric patients. Thus in one embodiment the present invention relates to said liquid nutritional composition comprising said mixture of non-digestible carbohydrates for use in paediatric patients and/or to be administered to paediatric patients and/or for providing nutrition to paediatric patients and/or for providing nutritional support to paediatric patients.

Also the invention relates to a liquid enteral composition comprising a digestible carbohydrate component, a lipid component, a protein component, and a mixture of non-digestible carbohydrates, wherein the mixture of non-digestible carbohydrates comprises a) at least 5 wt.% beta-galacto-oligosaccharides based on total non-digestible carbohydrates; b) fructan; c) non-digestible alpha-glucan; and d) hemicellulose;.

It has advantageously been found that non-digestible carbohydrates, also named dietary fibres, have water-retaining capacity and stimulate gastrointestinal motility by increasing faeces volume, bacterial growth, and bacterial degradation products, thereby promoting colonic propulsion, and reducing transit time.

Thus beneficially on the one hand the present liquid nutritional composition comprises beta-galacto-oligosaccharides which stimulate the activity of intestinal bifidobacteria and the immune system in a similar way as human milk oligosaccharides do. On the other hand the present liquid nutritional composition comprises plant derived, fermentable soluble fiber fructan, which also stimulates bifidobacteria; the plant derived, usually insoluble, fermentable fiber comprises non-digestible alpha-glucan, which is especially suitable for formation of intestinal butyrate; and the plant derived insoluble, structural and slowly fermentable fiber hemicellulose. The latter three fibres are representative for fibers in an adult diet. This fibre mixture therefore is an optimal transitional mix for children with an age of 1 to 14 years, in particular paediatric patients.

Beneficially dietary fibres also have a laxative, anti-constipation effect. Furthermore, dietary fibre may have a beneficial effect on the immune system and/or on the gastro-intestinal health. Administration of the fibre mixture of the present invention preferably results 1) in an increased formation of short chain fatty acids (SCFA) and other organic acids, 2) a formation of SCFA and other organic acid along the entire colon, 3) a relative increase of acetate and lactate based on total organic acids formed and/or 4) a decreased amount of gas formed based upon non-digestible carbohydrates administered or on SCFA formed. A fibre mixture having these properties has an enhanced beneficial effect, exerts its beneficial effect along the entire colon, beneficially affects the intestinal microbiota (especially regarding bifidobacteria and lactic acid bacteria), and/or does not have unwanted side effects such as bloating, cramps, and/or flatulence.

### Paediatric population

Paediatric patients in the present invention relate to children and adolescent human subjects from 1 up to and including 14 years of age who are under medical supervision for a disorder. More particular the invention relates to paediatric patients requiring nutritional support.

Oral nutrition supplements are useful to improve the dietary intake of paediatric patients who are unable to meet their nutritional requirements with normal foods alone. Children who may benefit from provision of oral supplements include children with increased nutritional requirements and/or fluid restrictions due to various medical conditions, including congenital heart disease, chronic lung disease, cystic fibrosis, (athetoid) cerebral palsy, trauma, surgical conditions, and failure to thrive. Furthermore, oral supplements can be useful in children with mechanical gastro-intestinal tract dysfunction (e.g. children with oro-facial malformations, facial/jaw injuries or swallowing disorders), and in children who tire easily and/or lack appetite due to their disease. Finally, oral supplements can be used as sole source of nutrition for the primary management of disease (eg inflammatory bowel disease).

Enteral tube feeding is the preferred method for meeting the nutritional requirements of a child who has some degree of gastro-intestinal function, but is unable to achieve adequate oral intake to meet the needs for growth and development. Indications for enteral tube feeding in children include incapacity or limited ability to eat (e.g. due to suck-swallow dysfunction), inability to meet requirements by oral intake (e.g. due to anorexia, increased metabolic needs), increased nutritional losses (e.g. due to impaired indigestion and/or absorption), altered metabolism (e.g. inborn errors of fasting adaption), and primary disease management (e.g. Crohn's disease).

Liquid clinical enteral nutrition designed for paediatric subjects differs from that for adult patients, based upon the difference in nutritional needs.

### Liquid enteral composition

The present composition is a liquid. Preferably the present composition is a ready-to-feed composition. Preferably the composition is administered orally, more preferably via a straw, or via a tube. Suitably, the composition is in a powdered form, which can be reconstituted with water to form a liquid, or in a liquid concentrate form, which should be diluted with water. When the composition is in a liquid form, or reconstituted to its liquid form, the preferred volume administered on a daily basis is in the range of about 100 to 2500 ml, more preferably about 200 to 2000 ml per day.

Preferably the composition is in a liquid form, with a viscosity of 1 to 100 mPa.s, more preferably of 2 to 60 mPa.s, even more preferably 2 to 40 mPa.s as measured using a Physica Rheometer MCR 300 (Physica Messtechnik GmbH, Ostfilden, Germany) at shear rate of 95 s⁻¹ at 20 °C. Such a low viscosity enables an easy and fast flow through a straw or a tube and hence a proper administration of the liquid composition. Furthermore, a low viscosity results in a normal gastric emptying and a better energy intake, which is essential for paediatric patients, especially when suffering from malnutrition, for optimal growth and development. The present invention also relates to a packaged power composition wherein said package is provided with instructions to admix the powder with a suitable amount of liquid, thereby resulting in a liquid composition with a suitable viscosity, preferably a viscosity between 1 and 60 mPa.s.

The present composition preferably comprises digestible carbohydrate and/or protein. The present composition more preferably comprises lipid, digestible carbohydrate and/or protein. The present composition is particularly suitable for providing the daily nutritional requirements to a child with the age of 1 to 14 years. The lipid preferably provides 20 to 55% of the total calories, the protein preferably provides 5 to 15% of the total calories and the digestible carbohydrate preferably provides 30 to 75% of the total calories of the composition. Preferably the present composition comprises lipid providing 25 to 50 % of the total calories, protein providing 6 to 13% of the total calories and digestible carbohydrate providing 40 to 65% of the total calories of the composition. More preferably the present composition comprises lipid providing 35 to 50 % of the total calories, protein providing 9 to 13% of the total calories and digestible carbohydrate providing 45 to 55% of the total calories of the composition. In order to meet the caloric requirements of the child, the composition preferably comprises 40 to 200 kcal per 100 ml, more preferably 75 to 175 kcal/100 ml, even more preferably 100 to 150 kcal/100 ml. This caloric density ensures an optimal ratio between water and calorie consumption. The amount of calories is the sum of the calories provided by the protein, the lipid, and the digestible carbohydrate.

The composition preferably comprises 0 to 10 g lipid per 100 ml, more preferably 2 to 8 g per 100 ml, more preferably 4 to 7 g per 100 ml. The present composition preferably comprises 20 to 55% lipid, more preferably 25 to 50%, more preferably 35 to 50% of the total calories of the composition.

The amount of saturated fatty acids is preferably below 45 wt.% based on total lipids more preferably below 25 wt.%. The concentration of monounsaturated fatty acids preferably ranges from 30 to 65% based on weight of total fatty acids. The concentration of polyunsaturated fatty acids preferably ranges from 15 to 60% based on weight of total fatty acids. Preferably the composition comprises the n-6 polyunsaturated fatty acid linoleic acid (LA) and the n-3 polyunsaturated fatty acid α-linolenic acid (ALA). LA and ALA are essential fatty acids and important for healthy growth and development of children. Preferably the weight ratio LA/ALA is between 4 and 10 more preferable between 5 and 7. Preferably the composition comprises long chain poly-unsaturated fatty acids (LC-PUFA). LC-PUFA are defined in the present invention as fatty acids or acyl chains with two or more double bonds and a chain length of 20 or above. Preferably the composition comprises docoshexaenoic acid (DHA) and/or eicosapentaenoic acid (EPA). DHA and EPA are n-3 LC-PUFA which are important for adequate neural development and cognitive function in children. Absence of dietary n-3 LC-PUFA may result in a low n-3 LC-PUFA status, because of a low metabolic ability to convert ALA to n-3 LC-PUFA in some paediatric subgroups. Furthermore, the presence of LC-PUFA improves the immune function, decreases inflammation and/or increases gut barrier integrity, which is advantageous for paediatric subjects. Preferably the composition comprises arachidonic acid (ARA). Preferably the composition comprises 5 to 1000 mg DHA and EPA per 100 ml, more preferably 10 to 800, even more 50 - 300 mg per 100 ml.

The composition preferably comprises 0.5 to 8 g protein per 100 ml, more preferably 2.0 to 6.5 g per 100 ml. Protein is to be taken as the sum of proteins, peptides and free amino acids. The amount of protein can be calculated according to the amount of nitrogen X 6.25. The protein preferably provides 5 to 15%, more preferably 6 to 13% even more preferably 9 to 13% based on total calories of the composition.

The present composition preferably comprises casein and/or whey proteins. Preferably the weight ratio casein:whey protein is 0:100 to 100:0, more preferably 10:90 to 90:10, more preferably 20:80 to 80:20. Preferably the composition comprises whey protein, more preferably 35 to 60 wt.% based on total protein. Whey protein advantageously results in better tolerance of feeds, an increased rate of gastric emptying, reduced gastro-oesophagal reflux (GOR) and/or reduced emesis. Food intolerance, slow gastric emptying, GOR, and emesis are particularly present in paediatric subject, more particularly children with neurological disorders. Whey proteins furthermore have the nutritional advantage that it has a better amino acid prophile. The composition may optionally comprise hydrolysed proteins and/or free amino acids. Preferably the composition does not comprise hydrolysed proteins and/or free amino acids, since this increases the osmotic load of the composition which is undesirable.

The composition preferably comprises 5 to 37 g digestible carbohydrates per 100 ml, more preferably 10 to 20 g per 100 ml. Preferably the digestible carbohydrate preferably provides 30 to 95%, more preferably 40 to 75% even more preferably 35 to 60 % of the total calories of the composition.

Preferably the composition comprises at least one digestible carbohydrate selected from the group consisting of lactose, maltodextrin, digestible starch, saccharose, glucose, and maltose. Preferably the present composition comprises maltodextrin and/or digestible starch. Using digestible carbohydrates with a higher degree of polymerization instead of mono- and disaccharides reduces the osmotic load, which is advantageous.

Preferably the composition does not comprise yeast, such as *Saccharomyces cerevisiae.* The presence of yeast adversely affects the taste and/or stability of the product. Preferably the composition comprises vitamins, minerals and trace elements in recommended daily amounts as known in the art.

The osmolarity of the present composition is preferably between 150 and 700 mOsmol/l, more preferably 200 to 400 mOsmol/l. This osmolarity advantageously reduced gastro-intestinal stress, results in an optimal balance between water and nutrient uptake, which is beneficial for paediatric and/or constipated children.

### Fibre mixture

The present composition comprises a mixture of beta-galacto-oligosaccharides, fructan, non-digestible alpha-glucan and hemicellulose. The present composition preferably further comprises galacturonic acid oligosaccharides, pectin and/or pectin degradation products. The present composition preferably further comprises cellulose. The present composition preferably further comprises a soluble non-digestible carbohydrate selected from the group consisting of arabinogalactan, glucomannan and galactomannan, preferably arabinogalactan. More preferably the composition is a mixture of beta-galacto-oligosaccharides, fructan, non-digestible alpha-glucan, hemicellulose, cellulose and soluble arabinogalactan. Preferably the composition comprises beta-galacto-oligosaccharides, fructan, non-digestible alpha-glucan, and hemicellulose in a weight ratio of 1 : (0.04 to 1) : (0.01 to 2) : (0.1 to 2). This ratio of fibres ensures an optimal balance and/or interaction between the different types of fibers and their specific beneficial effect.

Non-digestible carbohydrates are carbohydrates that are resistant to digestion and absorption in the human small intestine and enter the colon intact. So, compounds like lactose, maltose, glucose, standard maltodextrin and standard starch are regarded as digestible. The term "soluble" as used herein, when having reference to a non-digestible carbohydrate, means that the substance is water soluble according to the method described by L. Prosky et al., J. Assoc. Off. Anal. Chem. 71, 1017-1023 (1988). The term "fermentable" as used herein refers to the capability to undergo (anaerobic) breakdown by micro-organisms in the lower part of the gastro-intestinal tract (e.g. colon) to smaller molecules, in particular short chain fatty acids and lactate. The fermentability may be determined by the method described in Am. J. Clin. Nutr. 53, 1418-1424 (1991).

Preferably the composition comprises at least 0.2 g non-digestible carbohydrates per 100 ml composition, more preferably at least 0.5 g, even more preferably at least 0.75 g per 100 ml. Such quantities of non-digestible carbohydrates result in the advantageous effects of these non-digestible carbohydrates in the gastro-intestinal tract of the children. Preferably the composition comprises less than 15 g per 100 ml, more preferably less than 10 g per 100 ml, more preferably less than 5 g per 100 ml, even more preferably less than 2.5 g per 100 ml. Such high quantities of non-digestible carbohydrates are unsuitable for children and result in unwanted side effects such as bloating, abdominal pain, flatulence and/or a feeling of satiety. Preferably, for prevention purposes, the composition comprises between 0.2 and 2.5 g non-digestible carbohydrates per 100 ml. Preferably, for constipation treatment purposes the composition comprises between 1 and 10 g non-digestible carbohydrates per 100 ml. The amount of fiber can suitably be determined according to McCleary, 2007, Anal Bioanal Chem 389:291-308. This method suitably determines total fiber including resistant starch and non-digestible oligosaccharides.

### Beta-galacto-oligosaccharides

Beta-galacto-oligosaccharides as used in the present invention refers to oligosaccharides composed of over 50%, preferably over 65% galactose units based on monomeric subunits, with a degree of polymerization (DP) of 2 to 20, in which at least 50%, more preferably at least 75%, even more preferably at least 90%, of the galactose units are linked together via a beta-glycosidic linkage, preferably a beta-1,4-glycosidic linkage. The average DP is preferably of 3 to 6. A glucose unit may be present at the reducing end of the chain of galactose units. Beta-galacto-oligosaccharides are sometimes also referred to as transgalacto-oligosacchariodes (TOS). Beta-galacto-oligosaccharides can be analyzed according to AOAC method 2001.02. A suitable source of beta-galacto-oligosaccharides is Vivinal®GOS (commercially available from Borculo Domo Ingredients, Zwolle, Netherlands). Other suitable sources are Oligomate (Yakult), Cupoligo, (Nissin) and Bi2muno (Classado).

Preferably the composition comprises at least 0.05 g beta-galacto-oligosaccharides per 100 ml, more preferably at least 0.1 g, even more preferably at least 0.2 g, most preferably at least 0.4 g per 100 ml. Preferably the composition comprises at least 5 wt.% based on total non-digestible carbohydrates present in composition, more preferably at least 10 wt.%, more preferably at least 25 wt.%, even more preferably at least 30 wt.%. Beta-galacto-oligosaccharides are reminiscent to human milk oligosaccharides in that human milk oligosaccharides also comprise beta glycosidic linkages and comprise galactose as a monomeric unit. A high amount of beta-galacto-oligosaccharides is advantageous for paediatric patients and/or constipated children since it favourably stimulates the intestinal bifidobacteria, the intestinal production of the organic acids lactate and acetate and stimulates the immune system. The use of beta-galacto-oligosaccharides together with the other non-digestible carbohydrates of the invention results in an intestinal microbiota which is intermediate, regarding bifidobacteria, between infants and adult subjects.

Preferably the composition comprises less than 1.5 g beta-galacto-oligosaccharides per 100 ml, more preferably less than 1.0 g, even more preferably less than 0.8 g per 100 ml. Preferably the composition comprises less than 80 wt.% beta-galacto-oligosaccharides based on total non-digestible carbohydrates present in composition, more preferably less than 70 wt.%, even more preferably less than 55 wt.%. A too high amount of beta-galacto-oligosaccharides will result in an imbalance between beta-galacto-oligosaccharides with the other non-digestible carbohydrates of the invention. A too high amount of beta-galacto-oligosaccharides will result in a too fast and high fermentation in the beginning of the colon.

### Fructan

Fructan as used in the present invention refers to carbohydrates composed of over 50%, preferably over 65 % fructose units based on monomeric subunits, in which at least 50 %, more preferably at least 75%, even more preferably at least 90%, of the fructose units are linked together via a β glycosidic linkage, preferably a β 2,1 glycosidic linkage. A glucose unit may be present at the reducing end of the chain of galactose units. Fructan comprises levan, hydrolyzed levan, inulin, hydrolyzed inulin, fructo-oligosaccharides, fructo-polysaccharides, oligofructose and polyfructose. Preferably the composition comprises short chain fructo-oligosaccharides with an average chain length of 3 to 6, more preferably hydrolyzed inulin or synthetic fructo-oligosaccharide. Preferably the composition comprises long chain fructan with an average DP above 20, such as RaftilinHP. Preferably the composition comprises both short chain and long chain fructan. Preferably the weight ratio of short chain fructan to long chain fructan is from 0.1 to 10, more preferably from 1 to 10, even more preferably from 2.5 to 5. The presence of both short and long chain fructan advantageously results in fermentation from the beginning to the middle of the colon.

Preferably the composition comprises at least 0.03 g fructan per 100 ml, more preferably at least 0.05 g, even more preferably at least 0.1 g, most preferably at least 0.2 g per 100 ml. Preferably the composition comprises at least 4 wt.% based on total non-digestible carbohydrates present in composition, more preferably at least 8 wt.%, even more preferably at least 12 wt.%. A sufficient amount of fructan is advantageous for paediatric patients and/or constipated children since it favourably stimulates the intestinal bifidobacteria and/or the intestinal production of the organic acids. The use of fructan together with beta-galacto-oligosaccharides has a synergistic effect in respect to stimulation of bifidobacteria and production of organic acids. Preferably the weight ratio between beta-galacto-oligosaccharides and long chain fructan is between 1 and 20, more preferably between 6 and 12. The use of fructan together with the other non-digestible carbohydrates of the invention results in an intestinal microbiota which is intermediate, regarding bifidobacteria, between infants and adult subjects.

Preferably the composition comprises less than 1.5 g fructan per 100 ml, more preferably less than 1.0 g, even more preferably less than 0.5 g per 100 ml. Preferably the composition comprises less than 50 wt.% based on total non-digestible carbohydrates present in composition, more preferably less than 45 wt.%, even more preferably less than 30 wt.%. A too high amount of fructan will result in an imbalance between fructans with the other non-digestible carbohydrates of the invention. A too high amount of fructan will result in excessive gas formation, bloating and flatulence. Fructan can be analyzed according to AOAC method 997.08. A suitable source of fructan is RaftilinHP and RaftiloseP95 (commercially available from Orafti). Other suitable sources are RaftilinST (Orafti), Frutafit, Frutalose (Sensus), Fibrulin, Fibrulose (Cosucra), Actilight (Beghin-Meiji).

### Non-digestible α-glucan

The present composition comprises non-digestible alpha-glucan. Non-digestible alpha-glucan relates to carbohydrates polymers made up of at least 80 % glucose monomers, based on total monomers, preferably at least 85, which are bound together for more than 50% via alpha-glycoside linkages, and which escape digestion in the upper part of the gastro-intestinal tract and enter the colon.

Preferably the composition comprises resistant starch. Resistant starch can be determined according to McCleary & Monaghan (2002) J. AOAC Int 85, 665-675. Resistant starch is the starch that escapes digestion in the stomach and small intestine. Resistant starch includes starch that is physically inaccessible to the digestive enzymes in the stomach and small intestine, starch that occurs in its natural granular form (such as uncooked potato starch, green banana flour and high amylase corn), starch that is formed when starch-containing foods are cooked and cooled (retrograded starch), and starch that is chemically modified to resist digestion. Preferably the resistant starch is retrograded starch and/or starch that is chemically modified to resist digestion, most preferably retrograded starch, since this gives a more stable product. A suitable source of resistant starch is Novelose 330.

Preferably the composition comprises non-digestible dextrin. The terms "non-digestible dextrin", "indigestible polydextrin", "indigestible dextrin", "indigestible maltodextrin", "polydextrose", "resistant polydextrin", "resistant maltodextrin", "pyrodextrin" or "resistant dextrin" are used interchangeably in the present invention and refer to non-digestible carbohydrates which have a DP of 3 to 50, preferably of 4 to 20 and in which the monomeric units are at least 80 %, preferably at least 85 % originating from glucose (based on the total of monomeric units present), and comprising at least 50%, preferably at least 80% alpha glycosidic linkages. The average degree of polymerization is preferably between 10 and 16 monosaccharide units per molecule. In a preferred embodiment, the indigestible polydextrins are randomly branched and comprise α(1,4), α(1,6) glycosidic linkages. Additionally up to about 11% other monomers such as sorbitol and citric acid may be present. Indigestible dextrin can be obtained by heating and α-amylase treatment of starch. Alternatively, polydextrose can be obtained by heating (under vacuum) dextrose with a food acid catalyst and sorbitol and purifying the resulting water-soluble polymer. Indigestible dextrin is for example available under the tradename "Fibersol 2®" from Matsutami Inductries or Litesse® from Danisco. Suitable method to determine the non-digestible glucan prepared by vacuum thermal polymerization of glucose, using food acid and sorbitol (eg plydextrose, litesse) is AOAC method 2000.11. A suitable way to determine the non-digestible glucan derived by heat and enzyme treatment of starch (eg fibersol, pyrodextrin) is AOAC method 985.29.

Preferably the composition comprises at least 0.005 g non-digestible alpha-glucan per 100 ml, more preferably more than 0.0075 g, even more preferably more than 0.01 g per 100 ml. Preferably the composition comprises at least 0.5 wt.% based on total non-digestible carbohydrates present in composition, more preferably at least 0.8 wt.%, even more preferably at least 2 wt.%. A sufficient amount of non-digestible alpha-glucan is advantageous for paediatric patients and/or constipated children since it advantageously results in a fermentation at the more distal part of the colon. The use of non-digestible alpha-glucan advantageously results in the formation of butyrate, an organic acid which is a substrate of the intestinal enterocytes. The use of non-digestible alpha-glucan together with the other non-digestible carbohydrates of the invention results in an intestinal microbiota which is intermediate, regarding bifidobacteria, between infants and adult subjects.

Preferably the composition comprises less than 1.5 g non-digestible alpha-glucan per 100 ml, more preferably less than 1.0 g, even more preferably less than 0.8 g per 100 ml. Preferably the composition comprises less than 20 wt.% non-digestible alpha-glucan based on total non-digestible carbohydrates present in composition, more preferably less than 15 wt.%, more preferably less than 10 wt.%, even more preferably less than 5 wt.%. A too high amount of non-digestible alpha-glucan will result in an imbalance with the other non-digestible carbohydrates of the invention. A too high amount of non-digestible alpha glucan, especially resistant starch, may result in unfavourable product characteristics such as a high viscosity and precipitations.

### Hemicellulose

The present composition comprises hemicellulose. Hemicellulose in the present invention relates to insoluble, non-digestible carbohydrates derived from plants, excluding cellulose. Preferably the hemicellulose has a degree of polymerization is of 50 to 500 and is a branched polymer. Preferably hemicellulose comprises xylose, glucose, mannose, galactose, rhamnose, and arabinose as monomers. Preferably the most abundant monomer in hemicellulose is xylose. Hemicellulose preferably has a random, amorphous structure with little strength. Hemicelluloses include xylan, glucuronoxylan, arabinoxylan, and xyloglucan and insoluble arabinogalactan like fibres. Preferred sources of hemicellulose are legumes (soy, lentils, peas, beans) and cereals (corn, wheat, oat, rice). A suitable source of hemicellulose is soy polysaccharides (Fibrim 2000, Rettenmaier & Sohne).

Preferably the composition comprises at least 0.01 g hemicellulose per 100 ml, more preferably 0.02 g, even more preferably at least 0.05 g, most preferably at least 0.1 g per 100 ml. Preferably the composition comprises at least 1 wt.% hemicellulose based on total non-digestible carbohydrates present in composition, more preferably at least 4 wt.%, even more preferably at least 8 wt.%. A sufficient amount of hemicellulose is advantageous for paediatric patients and/or constipated children since it is very slowly fermentable and results in an even more prolonged fermentation towards the end of the colon. Furthermore, hemicellulose binds water and therefore will increase stool consistency. The use of hemicellulose together with the other non-digestible carbohydrates of the invention results in an intestinal microbiota which is intermediate, regarding bifidobacteria, between infants and adult subjects.

Preferably the composition comprises less than 2 g hemicellulose per 100 ml, more preferably less than 1 g, even more preferably less than 0.5 g per 100 ml. Preferably the composition comprises less than 60 wt.% hemicellulose based on total non-digestible carbohydrates present in composition, more preferably 50 wt.%, even more preferably less than 30 wt.%. A too high amount of hemicellulose will result in an imbalance with the other non-digestible carbohydrates of the invention and/or a too high water holding capacity. A too high amount of hemicellulose will result in unfavourable product characteristics regarding viscosity.

Hemicellulose can suitable be determined by subtracting the acid detergent fibre (determined according to method AOAC 973.18) from the enzyme modified neutral detergent fibre (determined according to method AOAC 2002.04).

In one embodiment the present invention concerns a liquid enteral composition comprising digestible carbohydrate, lipids, protein, and at least 0.2 g/100 ml of a mixture of non-digestible carbohydrates, wherein the mixture of non-digestible carbohydrates comprises a) at least 10 wt.%, preferably 10-80 wt.%, beta-galacto-oligosaccharides based on total non-digestible carbohydrates, b) at least 4 wt.%, preferably 4-30 wt.%, fructan based on total non-digestible carbohydrates, c) at least 0.5 wt.%, preferably 0.5-20 wt.%, non-digestible alpha-glucan based on total non-digestible carbohydrates and at least 1 wt.%, preferably 1-60 wt.%, hemicellulose based on total non-digestible

### Other non-digestible carbohydrates

In an embodiment, the present composition further comprises galacturonic acid oligosaccharides. The term galacturonic acid oligosaccharide as used in the present invention refers to an oligosaccharide wherein at least 50 mol% of the monosaccharide units present in the oligosaccharide is one selected from the group consisting of galacturonic acid. The galacturonic acid oligosaccharides used in the invention are preferably prepared from degradation of pectin, pectate, and/or polygalacturonic acid. Preferably the degraded pectin is prepared by hydrolysis and/or beta-elimination of fruit and/or vegetable pectins, more preferably apple, citrus and/or sugar beet pectin, even more preferably apple, citrus and/or sugar beet pectin degraded by at least one lyase.

In a preferred embodiment, at least one of the terminal galacturonic acid units of the galacturonic acid oligosaccharide has a double bond. The double bond effectively protects against attachment of pathogenic bacteria to intestinal epithelial cells. This is advantageous for paediatric patients. Preferably one of the terminal galacturonic acid units comprises a C₄-C₅ double bond. The galacturonic acid oligosaccharide can be derivatised. The galacturonic acid oligosaccharide may be methoxylated and/or amidated. In one embodiment the galacturonic acid oligosaccharides are characterized by a degree of methoxylation above 20%, preferably above 50% even more preferably above 70%. Preferably the composition comprises the beta-galacto-oligosaccharide, fructan and a pectin degradation product. The weight ratio beta-galacto-oligosaccharide : fructan : pectin degradation product is preferably (20 to 2) : 1 : (1 to 20), more preferably (12 to 7) : 1 : (1 to 3). Examples of, detection, measurement and analyses of the galacturonic acid oligosaccharides are given in WO 0/160378. Preferably the composition comprises at least 0.01 g galacturonic acid oligosaccharide per 100 ml, more preferably 0.02 g, even more preferably at least 0.04 g per 100 ml. Preferably the composition comprises at least 1 wt.% galacturonic acid oligosaccharide based on total non-digestible carbohydrates present in composition, more preferably at least 2 wt.%, even more preferably at least 5 wt.%. A sufficient amount of galacturonic acid oligosaccharide is advantageous for paediatric patients. The use of galacturonic acid oligosaccharide together with the other non-digestible carbohydrates of the invention results in an intestinal microbiota which is intermediate, between infants and adult subjects. Preferably the composition comprises less than 2 g galacturonic acid oligosaccharide per 100 ml, more preferably less than 1 g, even more preferably less than 0.5 g per 100 ml. Preferably the composition comprises less than 20 wt.% galacturonic acid oligosaccharide based on total non-digestible carbohydrates present in composition, more preferably 15 wt.%, even more preferably less than 10 wt.%. A too high amount of galacturonic acid oligosaccharide will result in an imbalance with the other non-digestible carbohydrates of the invention.

Preferably the present composition further comprises cellulose. Cellulose in the present invention relates to a preferably non-branched polymer of glucose molecules at least 90% bound to each other via beta-1,4-glycosidic linkages. Typically the degree of polymerization is above usually above 2000. Typically, cellulose is insoluble and hardly fermentable.

Preferably the composition comprises at least 0.01 g cellulose per 100 ml, more preferably at least 0.02 g, even more preferably at least 0.05 g, most preferably at least 0.1 g per 100 ml. Preferably the composition comprises at least 1 wt.% cellulose based on total non-digestible carbohydrates present in composition, more preferably at least 4 wt.%, even more preferably at least 8 wt.%. A sufficient amount of cellulose is advantageous for paediatric patients and/or constipated children since it has a high water holding capacity, thereby softening the stool. The use of cellulose together with the other non-digestible carbohydrates of the invention results in an intestinal microbiota which is intermediate, regarding bifidobacteria, between infants and adult subjects. The presence of cellulose will stabilize the liquid composition.

Preferably the composition comprises less than 2 g cellulose per 100 ml, more preferably less than 1 g, even more preferably less than 0.5 g per 100 ml. Preferably the composition comprises less than 60 wt.% cellulose based on total non-digestible carbohydrates present in the composition, more preferably less than 50 wt.%, even more preferably less than 30 wt.%. A too high amount of cellulose will result in an imbalance with the other non-digestible carbohydrates of the invention. A too high amount of cellulose will result in a too high water holding capacity and/or unfavourable product characteristics. A suitable source of cellulose is Vitacell. Preferably the composition comprises at least 0.01 g cellulose per 100 ml, more preferably 0.02 g, even more preferably at least 0.05 g, most preferably at least 0.1 g per 100 ml. Preferably the composition comprises at least 1 wt.% cellulose based on total non-digestible carbohydrates present in composition, more preferably at least 4 wt. %, even more preferably at least 8 wt. %. A sufficient amount of cellulose is advantageous for paediatric patients and/or constipated children, since cellulose binds water and therefore will increase stool consistency. Preferably the composition comprises less than 2 g cellulose per 100 ml, more preferably less than 1 g, even more preferably less than 0.5 g per 100 ml. Preferably the composition comprises less than 60 wt.% cellulose based on total non-digestible carbohydrates present in composition, more preferably 50 wt.%, even more preferably less than 30 wt.%. A too high amount of cellulose will result in an imbalance with the other non-digestible carbohydrates of the invention and/or a too high water holding capacity. A too high amount of cellulose will result in unfavourable product characteristics regarding viscosity.

Preferably the composition further comprises soluble non-digestible carbohydrates selected from the group of arabinogalactan, glucomannan and galactomannan, preferably arabinogalactan. Partially hydrolyzed galactomannan in combination with non-digestible alpha-glycan or fructan was found to have a synergistic effect regarding the fermentation by microbiota to short chain fatty acids. Suitable sources of galactomannan are Benefiber®. Suitable sources of arabinogalactan are gum Arabic (or gum acacia, gum Senegal, turkey gum) and FiberAid® (Larex)

Preferably the composition comprises at least 0.01 g arabinogalactan per 100 ml, more preferably at least 0.02 g, even more preferably at least 0.05 g, most preferably at least 0.1 g per 100 ml. Preferably the composition comprises at least 1 wt.% arabinogalactan based on total non-digestible carbohydrates present in composition, more preferably at least 4 wt.%, even more preferably at least 8 wt.%. A sufficient amount of arabinogalactan is advantageous for paediatric patients and/or (constipated) children since it results in an improved microbiota, increased water holding capacity and/or a fermentation throughout the colon. Furthermore, some types arabinogalactan was found to beneficially stimulate the immune system. The use of soluble arabinogalactan together with the other non-digestible carbohydrates of the invention results in an intestinal microbiota which is intermediate, regarding bifidobacteria, between infants and adult subjects.

Preferably the composition comprises less than 2 g arabinogalactan per 100 ml, more preferably less than 1 g, even more preferably less than 0.5 g per 100 ml. Preferably the composition comprises less than 60 wt.% arabinogalactan based on total non-digestible carbohydrates present in composition, more preferably less than 50 wt.%, even more preferably less than 30 wt.%. A too high amount of arabinogalactan will result in an imbalance with the other non-digestible carbohydrates of the invention. A too high amount of arabinogalactan will result in a unwanted product technological properties such as a high viscosity.

It was found that a fibre mixture comprising also arabinogalactan and cellulose beneficially increased the formation of short chain fatty acids and decreased the amounts of gas formed.

### Application

The present invention relates to liquid enteral nutrition intended for paediatric patients and/or constipated children. The mixture of non-digestible carbohydrates of the present invention beneficially affects the bowel health of children of 1 to 14 years. The mixture of non-digestible carbohydrates is fermented continuously along the entire colon, resulting in an improved intestinal microbiota and a continuous formation of short chain fatty acids. The composition of the present invention is therefore preferably used to treat and/or prevent constipation, to decrease the gastro-intestinal transit time, to decrease faecal consistency, to increase faecal output, and/or to increase gastro-intestinal motility, more preferably treat and/or prevent constipation in constipated children and/or paediatric patients. The formation of short chain fatty acids and the subsequent lowering of pH inhibits the growth of pathogenic intestinal micro-organisms. The composition of the present invention is preferably used for prevention of gastro-intestinal infections and/or diarrhoea in paediatric patients. The formation of short chain fatty acids results in the formation of mucus and/or food for enterocytes and hence in an increased gut barrier. The improvement of the microbiota may result in an improved immune system. The composition of the present invention is preferably used to prevent and/or treat infections, allergy, asthma and atopic dermatitis in paediatric pateints. The organic acids formed can be used by the body as an extra source of energy. The composition of the present invention is especially suitable to treat and/or prevent malnutrition in paediatric patients, more preferably in patients suffering from gastro-intestinal inflammation, more preferably suffering from Crohn's disease or ulcerative colitis. Preferably the composition is used for prevention and/or treatment of disease related malnutrition, diarrhoea and/or infections inflammation in paediatric patients. Also the present composition is for providing nutrition to paediatric patients. Also the present composition is for providing nutritional support to paediatric patients in need thereof, in particular to children of 1 to 14 years of age in need of nutritional support.

### EXAMPLES

### Example 1

Liquid, ready to drink sip feed intended for paediatric patients over 1 year of age, comprising per 100 ml:
150 kcal, 5.3 g protein (including casein and whey), 18.8 g digestible carbohydrates (including maltodextrin), 6.0 g fat (including vegetable oil, fish oil), 1.6 g fibre mixture. The composition further comprises minerals, trace elements, vitamins as known in the art. The composition further comprises 3 mg carnitine, 20 mg choline and 0.25 mg carotenoids per 100 ml and has an osmolarity of 390 mOsmol/l.

The fibre mixture comprises per g fibre:
0.45 g beta-galacto-oligosaccharides, 0.01 g resistant starch, 0.1 g non-digestible soy fiber (Fibrim 2000®, comprising about 81% hemicellulose including insoluble arabinogalactan)
0.08 g cellulose, 0.18 g fructan (0.05 g derived from Raftilin HP® and 0.13 gram derived from Raftilose P95®), 0.18 g soluble arabinogalactan.

### Example 2

Tube feeding for children of 1 to 6 years of age comprising per 100 ml:
100 kcal, 2.8 g protein (including casein and whey), 12.3 g digestible carbohydrates (including maltodextrin), 4.4 g fat (including vegetable and fish oil), 0.95 g fibre mixture. The composition further comprises minerals, trace elements, vitamins as known in the art, 2 mg carnitine, 20 mg choline, 7.5 mg taurine and has an osmolarity of 215 mOsmol/l The fibre mixture comprises per g fibre:
0.36 g beta-galactooligosaccharides, 0.01 g resistant starch, 0.126 g soy polysaccharide (Fibrim 2000®, comprising about 81% hemicellulose including insoluble arabinogalactan), 0.086 g cellulose, 0.15 g fructan (0.04 g derived from Raftilin HP® and
0.11 gram derived from Raftilose P95®) 0.19 g soluble soluble arabinogalactan and 0.072 g galacturonic acid oligosaccharides.

### Example 3

High energy tube feeding for children of 7 to 14 years of age comprising per 100 ml:
150 kcal, 4.9 g protein (including casein and whey), 18.5 g digestible carbohydrates (including maltodextrin), 6.3 g fat (including vegetable and fish oil) and 1.1 g fibre mixture of example 1. The composition further comprises minerals, trace elements, vitamins as known in the art, 4 mg carnitine, 43 mg choline, 15 mg taurine and has an osmolarity of 330 mOsmol/l.

### Example 4: Yogurt drink

A yoghurt drink with mixed dietary fibres (8 g/100 ml). The yoghurt drink comprises 3.0 g beta-galacto-oligosaccharides (source Vivinal GOS®), 3.0 g fructan (Frutafit TEX®, Cosun), 1.6 g soy fibre (Fibrim 2000®, J. Rettenmaier & Sohne, Ellwangen, Germany) and 0.33 g resistant starch 3 (Novelose 330, National Starch & Chemical GmbH, Neustadt, Germany) per 100 ml.

Energy/ml: about 65 kcal/100 ml, protein: about 3.2 g/100 ml, digestible carbohydrates: about 13.2 g/100 ml and lipids: about 0.05 g/100 ml.

### Example 5: Clinical trial

A randomised, double-blind, prospective controlled study with constipated children attending a paediatric clinic was performed. All children had to fulfil at least 2 out of 4 criteria of constipation: stool frequency less than 3 times per week, faecal incontinence 2 or more times per week, periodic passage of very large amounts of stool at least once every 7-30 days, or a palpable abdominal or rectal mass. Children aged 1 to 13 years were included. Patients received either the yoghurt drink (hereafter: fibre group) according to example 4 or, as a control, a yoghurt drink comprising lactulose (8 g/100 ml; hereafter lactulose group). After a baseline period of 1 week, patients were treated for 8 weeks followed by 4 weeks of weaning. The amount of fibre and fluid intake depended on body weight. Patients with a weight less than 15 kg received 125 ml daily, those with a weight between 15 kg and 20 kg received 250 ml daily and those with a weight above 20 kg received 375 ml daily. Using a standardized bowel diary parents recorded defecation frequency, incontinence frequency, stool consistency, presence of abdominal pain and flatulence, necessity for step-up medication and dry weight of faeces as well as adverse effects and taste appreciation, during the treatment period.

Ninety-seven children completed the study. No significant differences were found in baseline characteristics of these children. After the treatment period defecation frequency was increased from 3 to 7 times/week in the fibre group and from 2.5 to 6 times/week in the lactulose group. The consistency of stools in the lactulose group significantly changed to a more softer stools after 3 and 8 weeks of intervention. In the fibre group, a trend toward a statistically significantly softer stools was observed in week 3, and significantly softer stools were observed in week 8. The percentage dry weight of faeces decreased significantly from week 0 to week 3 in the lactulose group (30.3% vs. 26.5%) but not in the fibre treated group (27.3% vs. 28.1%). Abdominal pain and flatulence scores were comparable between both groups. In 3 cases (1 in the fibre group and 2 in the lactulose group) the study yoghurt intake was decreased because of persistent diarrhoea. The necessity of step-up medication during the treatment period was slightly higher in the fibre group after 3 weeks, comparable after 8 weeks and slightly higher in the lactulose group after 12 weeks. Taste score at 4 weeks and 8 weeks in the fibre group was 8 and in the lactulose group 7.

These results demonstrate that a liquid nutritional composition comprising beta-galacto-oligosaccharides, fructan, non-digestible alpha glucan and hemicellulose effectively reduces constipation in children to a same extent as lactulose but with an advantageous more prolonged effect and advantageously without a high increase of faecal water content.

### Example 6: In vitro fermentation of fibre mixtures.

An *in vitro* semi-dynamic batch fermentation system was used, using fresh faeces obtained from healthy toddlers.

Experimental medium used was McBain & MacFarlane medium (Buffered peptone water 3.0 g/l, yeast extract 2.5 g/l, Tryptone 3.0 g/l, L-Cysteme-HCl 0.4 g/l, bile salts 0.05 g/l, K2HP04.3H20 2.6 g/l, NaHC03 0.2 g/l, NaCl 4.5 g/l, MgS04.7H20 0.5 g/l, CaCl2 0.228 g/l, FeS04 0.005 g/l).

Fresh faecal material was mixed with McBain & MacFarlane medium, which is representative for the intestinal environment, in a weight ratio of 1:5. At t = 0, 6 ml of the faecal suspension was mixed with the fibre mixtures and transferred into a dialysis tube in a 100 ml bottle filled with buffered dialysis medium (K2HP04.3H20 2.6 g/l, NaHC03 0.2 g/l, NaCl 4.5 g/l, MgS04.7H20 0.5 g/l, CaCl2 0.228 g/l, FeS04.7H20 0.005 g/l, pH 6.3). The bottle was closed and incubated at 37 °C.

The following fibre mixtures were tested:
Mixture 1 is a fibre mixture currently used in infant milk formula for infants up to 1 year.
The fibre mixture comprises per g fibre 0.900 g beta-galacto-oligosaccharides, 0.100 g fructan (derived from Raftilin HP®). This fibre mixture has shown to improve the intestinal flora and fermentation by the intestinal flora to become more comparable to the intestinal flora and fermentation by the intestinal flora observed in breast fed infants.
Mixture 2 is a fibre mixture currently used in tube feeds intended for adult patients. The fibre mixture comprises per g fibre: 0.111 g resistant starch, 0.301 g non-digestible soy fiber (Fibrim 2000®, comprising about 81% hemicellulose including insoluble arabinogalactan) 0.113 g cellulose, 0.231 g fructan (derived from Raftilin ST® and Raftilose P95®), and 0.244 g soluble arabinogalactan. The fibre mixture is representative for fibres consumed in a Western adult diet.
Mixture 3 is the fibre mixture as described in examples 4 and 5.
Mixture 4 is the fibre mixture as described in examples 1 and 3.
Mixture 5 is the fibre mixture as described in example 2.

Samples of 0.5 ml were taken from the dialysis tube and from the dialysis buffer with a hypodermic syringe after 24 and 48 h and stored at -18 °C. Experiments were performed in duplicate and all handlings were performed in an anaerobic cabinet. Lactate was determined enzymatically, using a lactic acid detection kit with D- and L-lactate-dehydrogenase (Raisio Diagnostics Spa, Rome, Italy). Short chain fatty acids were detected with gas chromatography: SCFA were extracted in MilliQ. 2-ethylbutyrate was used as an internal standard. Samples were analyzed on a capillary column (Restek Stabilwax DA 15m x 0.53 x 1.0 mri; ZB-FFAB 15mx0.13x1.0nm) with FID detector. The mobile phase was helium. SCFA is the sum of acetate, butyrate and propionate.

### Results and Conclusions:

The results are expressed as amounts of mmol lactate or SCFA formed per g of fibre and shown in Table 1.

**Table 1 Organic acids (in mmol/g fibre) formed upon fermentation of different fibre mixtures at t=24 and t=48 h.**

| Mixture | | Lactate | SCFA | Acetate | Propionate | Butyrate |
|---|---|---|---|---|---|---|
| 1 | t=24 | 4.56 | 7.10 | 6.96 | 0.14 | 0.00 |
| | t=48 | 6.59 | 23.28 | 15.87 | 0.28 | 0.03 |
| 2 | t=24 | 1.12 | 3.97 | 3.43 | 0.48 | 0.06 |
| | t=48 | 1.69 | 14.18 | 8.69 | 1.38 | 0.14 |
| 3 | t=24 | 3.87 | 5.30 | 5.14 | 0.15 | 0.01 |
| | t=48 | 6.07 | 18.64 | 12.96 | 0.38 | 0.01 |
| 4 | t=24 | 3.21 | 6.21 | 6.03 | 0.17 | 0.01 |
| | t=48 | 5.27 | 21.00 | 14.43 | 0.34 | 0.03 |
| 5 | t=24 | 3.33 | 5.42 | 5.26 | 0.16 | 0.00 |
| | t=48 | 5.49 | 18.87 | 13.03 | 0.41 | 0.01 |

The table shows that fibre mixture 3, 4, and 5 show an intermediate effect regarding lactate production and SCFA production, compared with mixture 1, representative for an infant formula mixture, and mixture 2, representative for a tube feed for adults. Furthermore, the pattern of SCFA observed of mix 4, 5 and 6 is intermediate of that observed with mixture 1, which is high in acetate, and low propionate and butyrate, as observed in faeces of breast fed infants, and of that observed with mixture 2, which is lower in acetate and higher in propionate and butyrate, as observed in adults faeces.

The results also show that the presence of beta-galactooligosaccharides is important to obtain high amounts of lactate. The presence of an indigestible alpha-glucan, and hemicellulose most likely is responsible for the amounts of butyrate and propionate formed. Fructan is included as this is considered to act synergistically with the beta-galactooligosaccharides. Long chain fructan furthermore is considered to extend the fermentation to more distal parts of the colon.

These results therefore demonstrate that the fibre mixtures of the present invention are very suitable for paediatric patients above 1 year of age.

## Claims

1. Use of a mixture of non-digestible carbohydrates, wherein the mixture of non-digestible carbohydrates comprises
a. beta-galacto-oligosaccharide;
b. fructan;
c. non-digestible alpha-glucan; and
d. hemicellulose;
for manufacture of a liquid nutritional composition for administration to children of 1 to 14 years old.

2. The use according to claim 1 wherein the liquid nutritional composition is for one selected from the group consisting of administration to children in need of nutrition, administration to children needing nutritional support, prevention and/or treatment of disease-related malnutrition, prevention and/or treatment of inflammation, diarrhoea and/or infections.

3. The use according to claim 1 or 2 wherein the liquid nutritional composition is for prevention and/or treatment of constipation.

4. The use according to any one of claims 1-3, wherein the composition is for paediatric patients.

5. The use according to any one of the preceding claims wherein the mixture further comprises cellulose and/or arabinogalactan.

6. The use according to any one of the preceding claims wherein the mixture further comprises galacturonic acid oligosaccharides and/or pectin degradation products.

7. The use according to any one of the preceding claims wherein the liquid nutritional composition comprises at least 0.2 g non-digestible carbohydrates based on 100 ml of the composition.

8. The use according to any one of the preceding claims wherein the mixture comprises beta-galacto-oligosaccharides, fructan, non-digestible alpha-glucan, and hemicellulose in a weight ratio of 1 : (0.04 to 1) ; (0.01 to 2) : (0.1 to 2).

9. The use according to any one of the preceding claims wherein the nutritional composition is a liquid with a viscosity of 2 to 60 mPa.s at 20 °C and at shear rate of 95 s⁻¹.

10. The use according to any one of the preceding claims wherein the liquid nutritional composition comprises digestible carbohydrate, protein, and lipid.

11. The use according to claim 10 comprising 5-15% protein, 30-75% digestible carbohydrates and 20-55% lipid based on total calories of the composition.

12. The use according to claim 10 or 11 wherein the protein comprises whey protein.

13. The use according to any one of claims 10-12, wherein the lipid comprises long chain polyunsaturated fatty acids.

14. A liquid enteral composition comprising digestible carbohydrate, lipids, protein, and a mixture of non-digestible carbohydrates, wherein the mixture of non-digestible carbohydrates comprises
a at least 5 wt.% beta-galacto-oligosaccharides based on total non-digestible carbohydrates;
b fructan;
c non-digestible alpha-glucan; and
d hemicellulose.

15. The composition according claim 14 further comprising cellulose and arabinogalactan (gum acacia).

16. The composition according to claim 14 or 15 further comprising galacturonic acid oligosaccharides.

17. Use of a composition according to any one of claim 14 to 16 for use in, in particular administration to, children of 1 to 14 years.
